# EUROPEAN PATENT SPECIFICATION

(11) **EP 1 885 666 B1**
(45) Date of publication and mention of the grant of the patent: **11.06.2014**
(21) Application number: 06744586.6
(22) Date of filing: 28.04.2006
(51) Int. Cl.: C05F 17/02

(54) **WASTE TREATMENT SYSTEM**
ABFALLBEHANDLUNGSSYSTEM
SYSTÈME DE TRAITEMENT DES DÉCHETS

(30) Priority: 13.05.2005 IT BO20050343
(43) Date of publication of application: 13.02.2008
(73) Proprietor: AMBIENTALIA S.R.L., 40050 Frazione Toscanella, Dozza (BO) (IT)
(72) Inventor: BRESSAN, Loris, I-40026 Imola (IT)
(74) Representative: Firmati, Leonardo
(86) International application number: PCT/IB2006/001048
(87) International publication number: WO 2006/120517

(56) References cited:
- WO-A-01/05729
- WO-A-92/12938
- WO-A-02/062497
- WO-A-03/104165
- CA-A1- 2 206 068
- DE-A1- 3 627 265
- DE-C1- 4 422 855
- DE-U1- 29 816 749
- FR-A1- 2 074 604
- US-A- 5 461 843
- US-A1- 2004 219 650

## Description

### TECHNICAL FIELD

The present invention relates to a waste treatment system.

### BACKGROUND ART

The problem of waste disposal or recycling has been particularly felt for some time. In particular, the biggest problems of organic waste are related to its high environmental impact.

With regard to this, new European regulations related to waste disposal indicate sanitation and composting as particularly valid solutions.

Composting is a natural form of organic waste disposal known for a long time, by means of which a product, compost, is made and used in agriculture as soil conditioner, as fertiliser or as cultivation substrate.

In particular, composting is a bioxidising exothermal process which occurs in controlled conditions and, by means of the action of micro organisms, leads to the production of water, carbon dioxide, heat and compost.

Another form of organic waste exploitation relates to the production of biogas by anaerobic fermentation. The produced biogas may be either converted into electricity and/or heat in a cogeneration system or be introduced directly into the gas network.

In order to exploit both the potentials of biogas and those of compost, the organic waste recycling process should envisage two consequential steps: a first step in which the organic waste is subjected to an anaerobic treatment for biogas formation, and a second step in which the waste is subjected to aerobic treatment for the preparation of compost.

From WO-A-02062497 a waste treatment system is known for making biogas and compost, which comprises a large flexible plastic bag.

As apparent, the passage from the first to the second step may entail a series of problems above all of environmental and logistic nature. Indeed, the transportation of waste from an anaerobic reactor to an aerobic reactor is particularly complex and, obviously, environmentally hazardous.

### DISCLOSURE OF INVENTION

It is the object of the present invention to make a system for the treatment of organic waste which allows to simply and cost-effectively implement a complete recycling process performed either in only aerobic or in combined aerobic and anaerobic conditions, without presenting logistic or environmental problems.

The object of the present invention is a waste treatment system characterised in that it comprises an impermeable material container adapted to accommodate the waste, and presenting at least one opening for introducing the waste, air-tight closing means of said opening, percolate introducing means within the impermeable material container, means for collecting the formed biogas, a covering layer made of adsorbing and transpiring material arranged at an upper portion of said container, and a recirculation system of the air within said container.

### BRIEF DESCRIPTION OF THE DRAWINGS

The following example is provided by way of nonlimiting illustration for better understanding of the invention with the help of the figures in the accompanying drawing, in which:
- figure 1 is a side section with schematised parts of a preferred embodiment of the system according to the present invention;

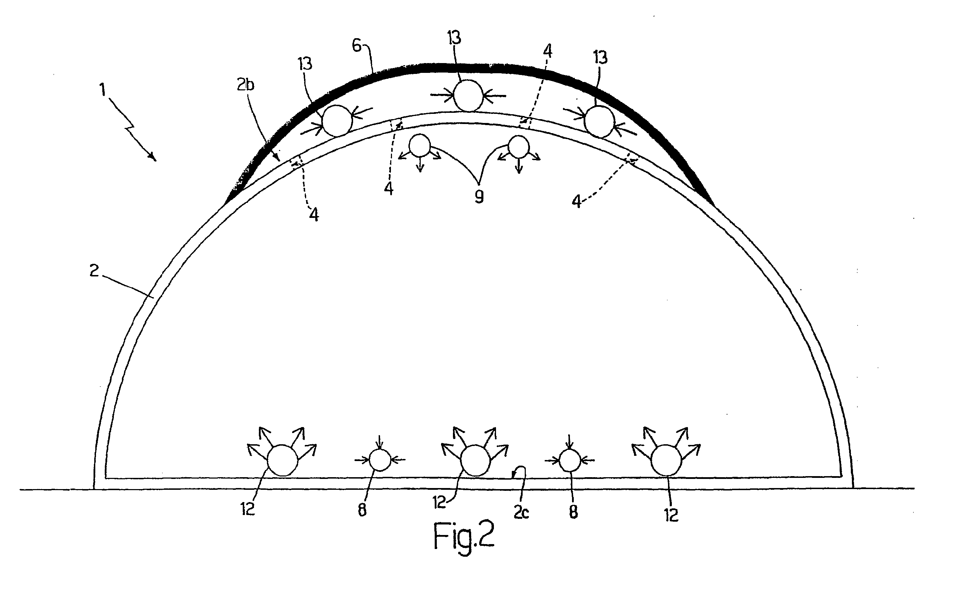

- figure 2 is a cross-section of the system shown in figure 1;
- figure 3 is a side section with schematised parts of a second embodiment of the system not belonging to the present invention; and
- figure 4 is a cross-section of the system shown in figure 3.

### BEST MODE FOR CARRYING OUT THE INVENTION

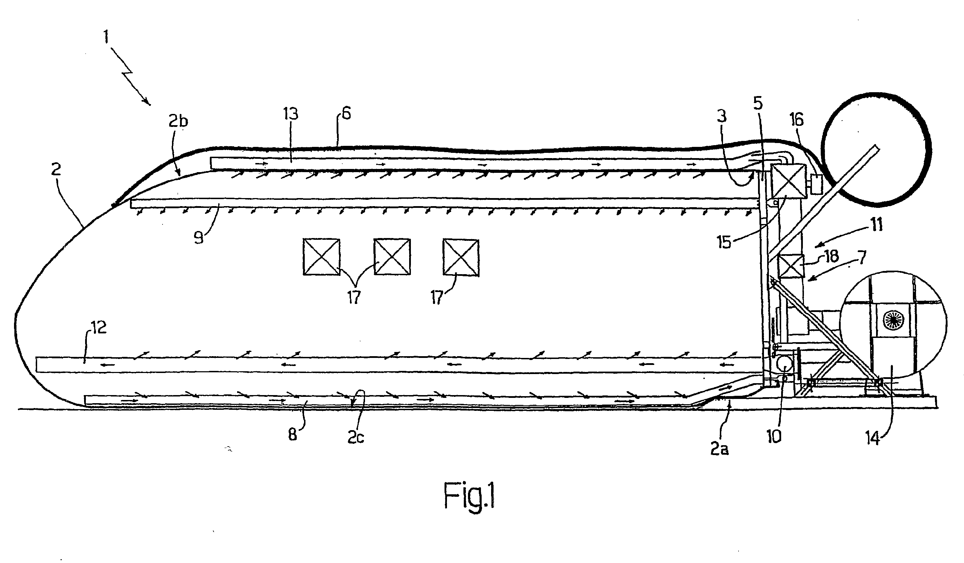

In the figure 1, it is shown as a whole by 1 the waste treatment system object of the present invention.

System 1 comprises a tubular container 2 made of polyethylene or other suitable material, presenting on one end 2a an opening 3 for introducing waste. The tubular container 2 is therefore made of non-rigid material for facilitating transportation, installation and removal thereof.

System 1 comprises closing means 5 (known and not described in general) for the opening 3, and an adsorbing and transpiring layer 6 arranged at the upper position 2b for closing the openings 4 formed as described below.

The adsorbing and transpiring layer 6 is formed by an open cell polyurethane matrix impregnated with activate carbon or other filtering system. The adsorbing and transpiring layer 6 has mainly the purpose of blocking odorous substances and filtering them before they are dispersed in the environment and of attenuating the output of bad odours caused by the decomposition of waste during composting. The function of the adsorbing and transpiring layer 6 is also to adsorb part of the output humidity to gradually give it to the decomposing material.

According to an alternative embodiment of the system according to the present invention, layer 6 does not present the features set forth above, and it is an impermeable material layer of the type with which the tubular container 2 is made.

System 1 comprises a recirculation system 7 of the percolate within the tubular container 2 for favouring the production of biogas during the anaerobic processing step.

The recirculation system 7 comprises a plurality of percolate collection tubes 8 arranged near a bottom 2c of the tubular container 2, a plurality of percolate introduction tubes 9 arranged near the upper portion 2b of the container 2, and a pump 10. Obviously, the tubes 8 and 9 are appropriately perforated.

In this way, the percolate inside the biomass can be aspirated by the tubes 8 by means of the action of the pump 10 and reintroduced within the biomass itself through tubes 9 to favour the production of biogas.

System 1 comprises a recirculation system 11 of air adapted to ensure appropriate oxygenation during the aerobic step.

The recirculation system 11 comprises a plurality of air intakes 12 arranged near a bottom 2c of the container 2, a plurality of air intake tubes 13 arranged between the upper portion 2b of the container 2 and the adsorbing and transpiring layer 6 and a fan 14 for recirculating the air. Obviously, tubes 12 and 13 are appropriately perforated.

During the aerobic treatment step, the intake tubes 13 have a two-fold purpose: the first is to take exhausted air from the waste mass being stabilised and to introduce it into the heap, the second is to ensure a closed-circuit system operation without the emission of gas and vapour outside.

System 1 comprises a device 15 (schematically shown in figure 1) for extracting air from outside the tubular container 2, which is connected to the air circulation system 11. Device 15 is provided with valve means (known and not shown), for connecting the external air intake to the recirculation system 11 or not according to the quantity of oxygen detected in the recirculation system 11 itself.

In particular, an oxygen sensor will check the percentage of oxygen present in the air circulating the recirculation system 11, and when the latter drops under a predetermined limit the valve means for mixing the exhaust air with the external air will be opened and the right oxygen mixture will be created.

Furthermore, the system 1 comprises a biogas collection device 16 (schematically shown in figure 1) connected to the air recirculation system 11 and adapted to collect the biogas formed following the anaerobic treatment step.

Finally, the system 1 comprises a plurality of thermometers 17 (schematically shown in figure 1) arranged within the tubular container 2, and a hygrometer 18 (schematically shown in figure 1) accommodated and operating in the recirculation system 11.

The system 1 comprises a control unit known and not shown which may be controlled via the Internet and which is capable of managing the various parts of the system.

In use, the waste to be processed, after being appropriately prepared by possible shredding and sieving, as well as mixed with appropriate structuring material, if required, is charged with inoculation liquid for boosting the fermentation process, and then introduced in the tubular container 2 by means of a typical bagging machine normally used for farming applications such as those used for bagging fodder and products intended for feeding animals.

Once the tubular container 2 is filled, the opening through which the waste was introduced is closed, the percolate recirculation system 7 is operated and the conditions for producing biogas, i.e. for performing the anaerobic process step, are created within the tubular container 2.

The anaerobic processing step lasts three to four weeks during which a biogas is produced that will be collected by device 16. The collected biogas will be subjected to a first pre-treatment step related to dehydration and purification and then will be introduced in the cogeneration plant.

After the first anaerobic processing step, openings 4 are made at the upper portion 2b. At this point, the most possible percolate which can be aspirated is aspirated from within the tubular container by tubes 8 and recovered in a tank, and the air recirculation system 11 is put into operation. In these conditions, the anaerobic processing step will last for approximately from two to three weeks.

During this aerobic process step, according to the treatment conditions, either the complete sanitation and bio-stabilisation of the waste heap or the formation of compost is obtained.

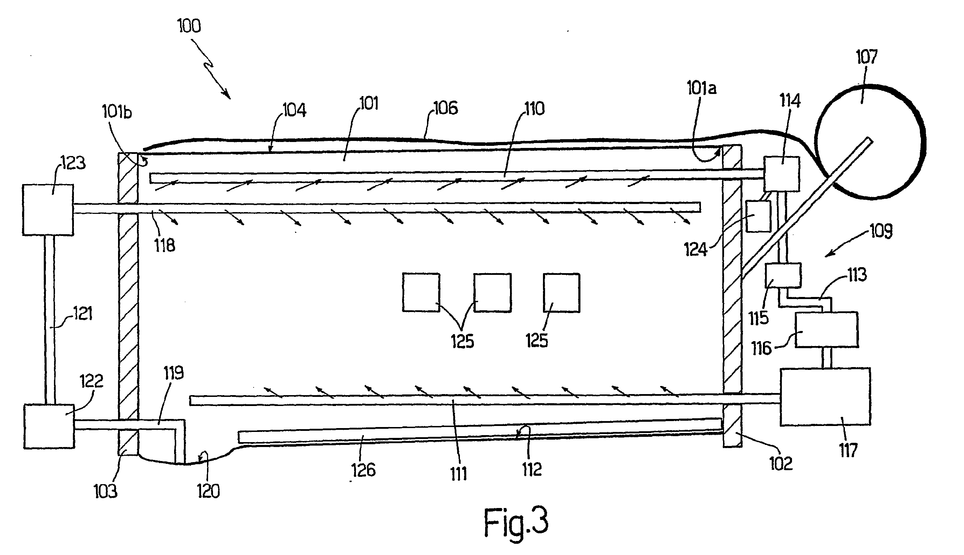

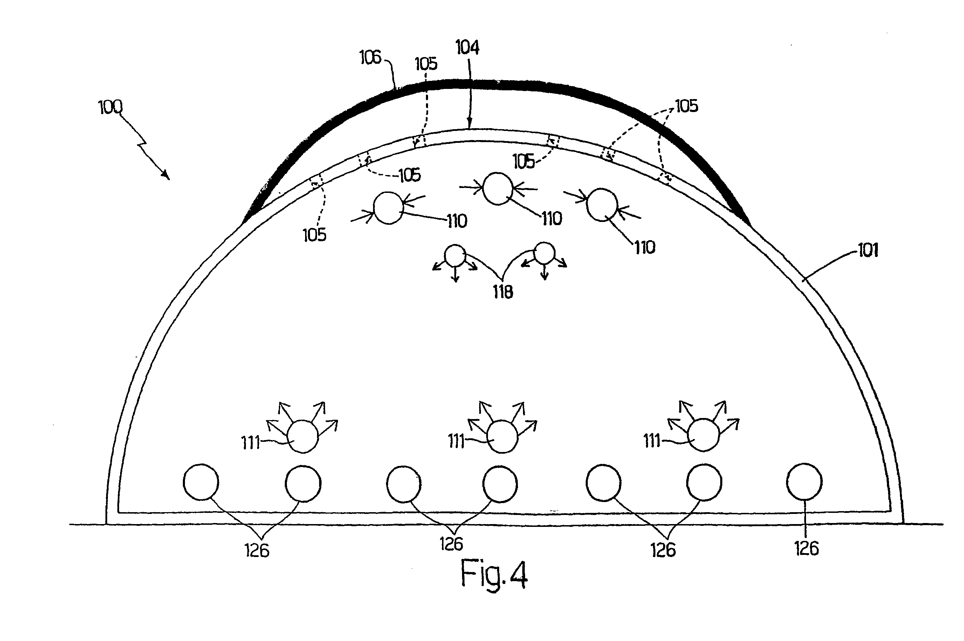

In figures 3 and 4, it is indicated by 100 a further embodiment of the system not belonging to the present invention. System 100 comprises a tubular container 101 made of polyethylene or other suitable material and presenting two ends 101a and 101b closed by respective plates 102 and 103 made of metallic material. As previously specified for system 1, the tubular container 101 is also made of non-rigid material which facilitates transportation, installation and removal thereof.

System 100 comprises an adsorbing and transpiring layer 106 arranged at an upper portion 104 of the tubular container 101 to close openings 105 formed as described below. Layer 106 has the same features already shown for layer 6 and is wound on specific winding/rolling means 107 fastened to the plate 102.

System 100 comprises an air recirculation system 109 comprising a plurality of air intake tubes 110 arranged within the tubular container 101 near the upper portion 104 and fastened to the plate 102, a plurality of air intakes 111 arranged within the tubular container 101 near a bottom portion 112 and also fastened to the plate 102 and a union line 113 fastened to the plate 102 externally to the tubular container 101 and adapted to join tubes 110 with tubes 111. The union line 113 comprises in sequence following the flow of air and schematically illustrated in figure 3, a device 114 for collecting air from the outside provided with valve means, an oxygen gauge 115, a dehumidifier 116 and a fan 117. Optionally, a cooling element may be arranged on union line 113 and downstream of the dehumidifier 116. As apparent, tubes 111 and 112 present a plurality of holes adapted to allow the passage of air.

System 100 comprises a percolate recirculation system comprising a plurality of perforated percolate introduction tubes 118 arranged within the tubular container 101 near its upper portion 104 and fastened to the plate 103, a plurality of collection tubes 119 arranged within the tubular container 101 fastened to the plate 103 and adapted to take by means of its end from within a collection tank 120, and a union line 121 fastened to the plate 103 externally to the tubular container 101 and adapted to join tubes 118 and tubes 119. The collection tank 120 is arranged in the bottom portion 112 of the tubular container 101 and collects the percolate formed due to the presence of a plurality of draining tubes 126 resting on the bottom portion 112 arranged on an incline. The union line 121 comprises (schematically shown in figure 3) a pump 122 and a union 123 adapted to be connected to an external percolate source to be introduced within the tubular container 101.

As described above for system 1, system 100 comprises (schematically shown in figure 3) a biogas collection device 124 connected with the air recirculation system 109 and a plurality of thermometers 125.

System 100 comprises a control unit (known and not shown) which may be controlled via the Internet and which is capable of reading the values shown by the measuring instruments and of consequently controlling the various parts of the system.

The waste treatment procedure using system 100 is the same as that shown with reference to system 1, with the difference that the percolate introduced within the tubular container may also be originated outside the system and that the air recirculation collection tubes are arranged inside instead of outside the container and falls therefore outside the scope of claim 1.

The system of the present invention offers the advantage that the entire process is performed in a single system thus avoiding movements of materials with respective environmental problems.

Moreover, the system of the present invention offers the advantage of using particularly economical consumable materials without for this loosing in efficiency, and allows to perform the processing processes without logistic constraints, minimising at the same time the leakage of gas and bad odours.

Another advantage offered by the system is that it may be mobile and therefore moved to various areas of the waste treatment system according to logistics.

Moreover, the system of the present invention allows to perform treatments also outdoor or sheltered only by a lean-to roof, thus eliminating the enormous costs for purifying air within specific sheds.

Finally, the system may be used also separately, i.e. either as an anaerobic system for the production of biogas or an aerobic system for stabilisation and compositing.

## Claims

1. A waste treatment system (1) comprising an impermeable tubular container (2) adapted to accommodate the waste, and presenting at least one opening (3) for introducing the waste, air-tight closing means (5) of said opening (3), percolate introducing means (9) within the impermeable tubular container (2), **characterised in that** it comprises means for collecting (16) the formed biogas, a covering layer (6) made of adsorbing and transpiring material or impermeable material and arranged at an upper portion (2b) of said container (2), the upper portion (2b) presenting openings (4) ; a recirculation system of the air (11) having a plurality of air intake tubes (13) arranged between said impermeable container (2) and said covering layer (6), and a plurality of air intakes (12) arranged within said container (2); a recirculation system (7 ) of the percolate within the tubular container (2) for favouring the production of biogas during an anaerobic processing step.

2. A system according to claim 1, **characterised in that** said impermeable tubular container (2) is made of non-rigid material and adapted to be gathered and spread according to the various steps of use.

3. A system according to claim **1, characterised in that** said air circulation system (11) comprises a device (15) for collecting air from outside the tubular container (2).

4. A system according to claim **3, characterised in that** said air collection device is provided with valve means adapted to be operated according to the quantity of oxygen measured in said recirculation system.

5. A system according to claim 1, **characterised in that** said recirculation system (7) comprises a plurality of percolate collection tubes (8) arranged near a bottom (2c) of the tubular container (2), a plurality of percolate introduction tubes (9) arranged near the upper portion (2b) of the container (2), and a recirculation pump (10).

## Patentansprüche

1. Abfallbehandlungssystem (1), umfassend einen undurchlässigen rohrförmigen Behälter (2), der dazu geeignet ist, den Abfall aufzunehmen, und aufweisend mindestens eine Öffnung (3) zum Einführen des Abfalls, Mittel (5) zum luftdichten Verschluss der Öffnung (3), Mittel (9) zum Einführen von Perkolat innerhalb des undurchlässigen rohrförmigen Behälters (2), **dadurch gekennzeichnet, dass** es Mittel (16) zum Sammeln des sich gebildeten Biogases, eine Deckschicht (6) aus adsorbierendem und transpirierendem Material oder undurchlässigem Material, die an einem oberen Abschnitt (2b) des Behälters (2) angeordnet ist, umfasst, wobei der obere Abschnitt (2b) Öffnungen (4) aufweist; ein System (11) zur Rezirkulation der Luft, aufweisend eine Vielzahl von Lufteinlassrohren (13), die zwischen dem undurchlässigen Behälter (2) und der Deckschicht (6) angeordnet ist, und eine Vielzahl von Lufteinlässen (12), die innerhalb des Behälters (2) angeordnet ist; ein System (7) zur Rezirkulation des Perkolats innerhalb des rohrförmigen Behälters (2) zur Förderung der Biogasproduktion während eines anaeroben Verarbeitungsschrittes.

2. System nach Anspruch 1, **dadurch gekennzeichnet, dass** der undurchlässige, rohrförmige Behälter (2) aus nicht-starrem Material ist und dazu geeignet ist, gemäß den verschiedenen Verwendungsschritten zusammengerafft und ausgebreitet zu werden.

3. System nach Anspruch 1, **dadurch gekennzeichnet, dass** das Luftzirkulationssystem (11) eine Vorrichtung (15) zum Sammeln von Luft von außerhalb des rohrförmigen Behälters (2) umfasst.

4. System nach Anspruch 3, **dadurch gekennzeichnet, dass** die Luftsammelvorrichtung mit Ventilmitteln ausgestattet ist, die dazu geeignet sind, gemäß der Sauerstoffmenge, die im Rezirkulationssystem gemessen wird, betrieben zu werden.

5. System nach Anspruch 1, **dadurch gekennzeichnet, dass** das Rezirkulationssystem (7) eine Vielzahl von Rohren (8) zum Sammeln von Perkolat, die in der Nähe eines Bodens (2c) des rohrförmigen Behälters (2) angeordnet ist, eine Vielzahl von Rohren (9) zum Einführen von Perkolat, die in der Nähe des oberen Abschnitts (2b) des Behälters (2) angeordnet ist, und eine Rezirkulationspumpe (10) umfasst.

## Revendications

1. Système de traitement des déchets (1) comprenant un conteneur tubulaire imperméable (2) conçu pour accueillir les déchets, et présentant au moins une ouverture (3) servant à introduire les déchets, des moyens de fermeture étanche à l'air (5) de ladite ouverture (3), des moyens d'introduction du lixiviat (9) à l'intérieur du conteneur tubulaire imperméable (2), **caractérisé en ce qu'**il comprend des moyens de collecte (16) du biogaz formé, une couche de couverture (6) constituée d'un matériau adsorbant et transpirant ou d'un matériau imperméable et disposée au niveau d'une partie supérieure (2b) dudit conteneur (2), la partie supérieure (2b) présentant des ouvertures (4); un système à recirculation d'air (11) disposant d'une pluralité de tube d'admission d'air (13) disposés entre ledit conteneur imperméable (2) et ladite couche de couverture (6), ainsi qu'une pluralité d'entrées d'air (12) disposées à l'intérieur du conteneur (2) ; un système à recirculation (7) du lixiviat à l'intérieur du conteneur tubulaire (2) pour favoriser la production de biogaz lors d'une étape de traitement anaérobique.

2. Système selon la revendication 1, **caractérisé en ce que** ledit conteneur tubulaire imperméable (2) est fabriqué dans un matériau mou et adapté pour être recueilli et étendu selon les différentes étapes d'utilisation.

3. Système selon la revendication 1, **caractérisé en ce que** ledit système à circulation d'air (11) comprend un dispositif (15) servant à recueillir l'air depuis l'extérieur du conteneur tubulaire (2).

4. Système selon la revendication 3, **caractérisé en ce que** ledit dispositif recueilleur d'air est pourvu de moyens à vanne conçus pour être actionnés selon la quantité d'oxygène mesurée dans ledit système à recirculation.

5. Système selon la revendication 1, **caractérisé en ce que** ledit système à recirculation (7) comprend une pluralité de tubes recueilleurs de lixiviat (8) disposés près d'un fond (2c) du conteneur tubulaire (2), une pluralité de tubes d'introduction de lixiviat (9) disposés près de la partie supérieure (2b) du conteneur (2), et une pompe de recirculation (10).
